# EUROPEAN PATENT APPLICATION

(11) **EP 2 213 286 A1**
(43) Date of publication of application: **04.08.2010**
(21) Application number: 09382015.7
(22) Date of filing: 02.02.2009
(51) Int. Cl.: A61K 31/196, A61K 31/245, A61K 31/44, A61K 8/41, A61P 3/00, A61P 3/04, A61P 3/06, A61P 3/10, A61P 9/10, C07D 213/79

(54) **N-(PHENYL OR PYRIDYL)PRENYLAMINE DERIVATIVES FOR THE TREATMENT OF OBESITY, DIABETES AND CARDIOVASCULAR DISEASES**

(71) Applicant: Zeltia, S.A., 28003 Madrid (ES)
(72) Inventor: Sanatamaría Núñez, Gema, E-28003, Madrid (ES); Martínez Díez, Marta, E-28003, Madrid (ES)
(74) Representative: ABG Patentes, S.L.

(57) **Abstract**

The invention relates to the use of a compound of formula (I): 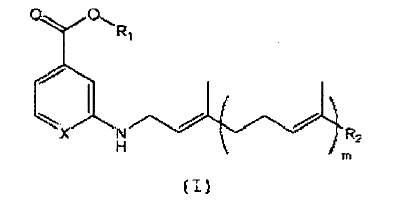 its pharmaceutically acceptable salts, and/or solvates, in the preparation of a medicinal product for the treatment of a disease presenting altered adiponectin levels or in which it is necessary to alter the adiponectin levels, in particular diabetes, obesity, metabolic syndrome, arteriosclerotic cardiovascular disease, dyslipidemia and lipodystrophy.

## Description

### FIELD OF THE INVENTION

The present invention relates to the use of compounds derived from N- (phenyl or pyridyl) prenylamine in the preparation of a medicinal product for the treatment of diseases or conditions presenting altered adiponectin levels or in which it is necessary to alter the adiponectin protein levels, such as diabetes, metabolic syndrome, arteriosclerotic cardiovascular disease, dyslipidemia or lipodystrophy.

### STATE OF THE ART

Adipose tissue is an important endocrine organ, secreting multiple metabolically active important proteins referred to as adipokines. Some known adipokines are leptin, tumor necrosis factor (TNF)-α, interleukin (IL)-6, adipsin and adiponectin. Most adipokines are secreted by adipocytes *per se*, although some can be secreted by other cell types in adipose tissue.

Adiponectin is a protein synthesized by white adipose tissue, circulating at relatively high plasma concentrations (2-30 µg/ml), it has an important role in glucose and lipid metabolism. Several studies have demonstrated the relationship between adiponectin and insulin sensitivity. A relationship between adiponectin and cardiovascular diseases has also been proven.

Adiponectin is a 247-amino acid protein (Mr 30 kDa) consisting of 4 domains. The first domain is a signal peptide located in the amino-terminal area allowing the secretion of the hormone outside the adipocytes; the second domain is a 28-amino acid region varying among species, the third domain is a collagenous domain formed by 22 glycine-X-tyrosine triplets; and finally a globular domain in the carboxy-terminal region.

Adiponectin molecules undergo a post-translational modification within the adipocyte, being grouped in multimeric forms, including trimers, hexamers and oligomers with a high molecular weight. Monomeric adiponectin is not usually detected in the blood flow.

Low plasma adiponectin levels are associated to adverse metabolic conditions, such as diabetes, metabolic syndrome, dyslipidemia, lipodystrophy and arteriosclerotic cardiovascular disease. It has been found that in these disorders there is also reduced adiponectin mRNA expression in plasma and adipose tissue; it has been considered that this can be due to an altered adipocyte functioning.

Unlike other adipokines, the concentration of which increases as the lipid mass increases, the circulating adiponectin levels are paradoxically reduced in obese individuals, the circulating adiponectin levels being inversely proportional to the body mass index (BMI) and to the body fat percentage (Arita Y et al., "Paradoxical decrease of an adipose-specific protein, adiponectin, in obesity", Biochem Biophys Res Commun 1999; 257 (1) : 79-83*;* Cnop M et al., "Relationship of adiponectin to body fat distribution, insulin sensitivity and plasma lipoproteins: evidence for independent roles of age and sex", Diabetologia 2003; 46 (4): 459-469*;* Kern PA et al., "Adiponectin expression from human adipose tissue: relation to obesity, insulin resistance, and tumor necrosis factor-alpha expression", Diabetes 2003; 52 (7): 1779-1785).

In addition, several studies have demonstrated a correlation between plasma adiponectin levels and insulin sensitivity (Berg AH et al., "The adipocyte-secreted protein Acrp30 enhances hepatic insulin action", Nat Med 2001; 7 (8): 947-953*;* Combs TP et al., "Induction of adipocyte complement-related protein of 30 kilodaltons by PPARgamma agonists: a potential mechanism of insulin sensitization", Endocrinology 2002; 143 (3): 998-1007*;* Hotta K et al., "Circulating concentrations of the adipocyte protein adiponectin are decreased in parallel with reduced insulin sensitivity during the progression to type 2 diabetes in rhesus monkeys", Diabetes 2001; 50 (5): 1126-1133*;* Steffes MW et al., "Serum adiponectin in young adults - interactions with central adiposity, circulating levels of glucose, and insulin resistance: the CARDIA study", Ann Epidemiol 2004; 14 (7): 492-498*;* Weyer C et al., "Hypoadiponectinemia in obesity and type 2 diabetes: close association with insulin resistance and hyperinsulinemia", J Clin Endocrinol Metab 2001; 86 (5): 1930-1935).

In fact, a reduction in adiponectin levels could identify a resistance to insulin before the development of diabetes *per se,* according to some studies (for example, Hotta K et al. mentioned above). Other studies have associated high adiponectin levels with a reduced risk of developing type two diabetes in multiple ethnic groups (Daimon M et al., "Decreased serum levels of adiponectin are a risk factor for the progression to type 2 diabetes in the Japanese Population: the Funagata study", Diabetes Care 2003; 26 (7): 2015-2020*;* Lindsay RS et al., "Adiponectin and development of type 2 diabetes in the Pima Indian population", Lancet 2002; 360 (9326): 57-58*;* Spranger J et al., "Adiponectin and protection against type 2 diabetes mellitus", Lancet 2003; 361 (9353): 226-228).

Reduced adiponectin levels are also associated to coronary heart disease (Hotta K et al. "Plasma concentrations of a novel, adipose-specific protein, adiponectin, in type 2 diabetic patients." Arterioscler Thromb Vasc Biol 2000; 20 (6): 1595-1599*;* Pischon T et al., "Plasma adiponectin levels and risk of myocardial infarction in men." JAMA 2004; 291 (14): 1730-1737). The second study has shown that subjects with high adiponectin levels had a significantly reduced risk of myocardial infarction. Several studies have studied the relationship between adiponectin and cardiovascular risk factors (Hotta K et al. "Plasma concentrations of a novel, adipose-specific protein, adiponectin, in type 2 diabetic patients." Arterioscler Thromb Vasc Biol 2000; 20 (6): 1595-1599*;* Shetty GK et al, "Circulating adiponectin and resistin levels in relation to metabolic factors, inflammatory markers, and vascular reactivity in diabetic patients and subjects at risk for diabetes" Diabetes Care 2004; 27 (10): 2450-2457*;* Mantzoros CS et al, "Circulating adiponectin levels are associated with better glycemic control, more favorable lipid profile, and reduced inflammation in women with type 2 diabetes", J Clin Endocrinol Metab 2005; 90 (8): 4542-4548*;* Schulze MB et al., "Relationship between adiponectin and glycemic control, blood lipids, and inflammatory markers in men with type 2 diabetes", Diabetes Care 2004; 27 (7): 1680-1687). These results suggest that adiponectin has an important role in atherosclerotic cardiovascular disease.

The administration of adiponectin to murine diabetes and lipoatrophy models has shown an improvement of insulin sensitivity. Both in wild-type mice and murine type 1 and type 2 diabetes models, the peritoneal injection of adiponectin caused a significant reduction of glucose levels (Berg AH et al., "The adipocyte-secreted protein Acrp30 enhances hepatic insulin action", Nat Med 2001, 7 (8): 947-953;). The same effect has been obtained by means of the systemic infusion of the adiponectin globular domain to animal obesity, diabetes and lipoatrophy models (Yamauchi T et al., "The fat-derived hormone adiponectin reverses insulin resistance associated with both lipoatrophy and obesity", Nat Med 2001, 7 (8): 941-946).

The effects of adiponectin overexpression have also been studied in animal models. Transgenic mice overexpressing globular adiponectin showed protection against resistance to insulin induced by a high-fat diet (Yamauchi T et al., "Globular adiponectin protected ob/ob mice from diabetes and ApoEdeficient mice from atherosclerosis", J Biol Chem 2003, 278 (4): 2461-2468). Other transgenic mice, with chronically high adiponectin levels, showed an increase in lipid clearance, as well as in the suppression of insulin -mediated endogenous glucose production (Combs TP et al., "A transgenic mouse with a deletion in the collagenous domain of adiponectin displays elevated circulating adiponectin and improved insulin sensitivity", Endocrinology 2004, 145 (1): 367-383). In another murine atherosclerosis model, it was shown that globular adiponectin can protect against atherosclerosis (Yamauchi T et al., "Globular adiponectin protected ob/ob mice from diabetes and ApoEdeficient mice from atherosclerosis", J Biol Chem 2003, 278 (4): 2461-2468).

In spite of the benefits shown in the direct administration of adiponectin to animal models, it would be complicated to administer adiponectin directly to human subjects due to the large protein structure of adiponectin, as well as due to need for its post-translational processing. Therefore, there is a need of achieving activation of adiponectin expression in the organism by means of administering compounds activating adiponectin expression. As an example of such compounds, several studies have shown that treatment with thiazolidinediones causes an increase in circulating adiponectin levels in human subjects. This has been observed with rosiglitazone (Combs TP et al., "Induction of adipocyte complement-related protein of 30 kilodaltons by PPARgamma agonists: a potential mechanism of insulin sensitization", Endocrinology 2002 143 (3): 998-1007; Tiikkainen M et al., "Effects of rosiglitazone and metformin on liver fat content, hepatic insulin resistance, insulin clearance, and gene expression in adipose tissue in patients with type 2 diabetes", Diabetes 2004, 53 (8): 2169-2176) as well as with pioglitazone (Hirose H et al., "Effects of pioglitazone on metabolic parameters, body fat distribution, and serum adiponectin levels in Japanese male patients with type 2 diabetes", Metabolism 2002, 51 (3) : 314-317) and with troglitazone (Phillips SA et al., "Modulation of circulating and adipose tissue adiponectin levels by antidiabetic therapy", Diabetes 2003, 52 (3): 667-674; Maeda N et al., "PPARgamma ligands increase expression and plasma concentrations of adiponectin, an adipose-derived protein", Diabetes 2001, 50 (9): 2094-2099; Yu JG et al., "The effect of thiazolidinediones on plasma adiponectin levels in normal, obese, and type 2 diabetic subjects", Diabetes 2002, 51 (10): 2968-2974).

The insecticidal activity of a particular family of N-(phenyl) prenylamine has been reported in Pinchin R et al., "Screening and Structure-Activity Relationships of Synthetic Juvenile Hormone Analogues for Panstrongylus megistus, a Primary Vector of Chagas' Disease in Brazil", J Econ Entomol 1978, 71 (6) : 950-955; and Oliveira AM et al., "Structure-Activity Relationships of 110 Candidate Juvenile Hormone Analogues for Panstrongylus megistus (Burmeister, 1835), a Vector of Chagas' Disease (Hemiptera, Reduviidae, Triatominae)", Rev Brasil Biol 1981, 41(1): 197-204. In said documents it is described how the pupae of *Panstrongylus megistus,* a bug responsible for the transmission of Chagas' disease, was juvenalized through topical treatment with said N-(phenyl) prenylamine. No medical or cosmetic application on humans is disclosed.

### SUMMARY OF THE INVENTION

The object of the present invention is to provide compounds activating adiponectin expression, enabling its use in preparing medicinal products for the treatment of diseases such as diabetes, obesity, metabolic syndrome, arteriosclerotic cardiovascular disease, dyslipidemia and lipodystrophy.

According to a first aspect, the present invention relates to the use of a compound of formula (I): 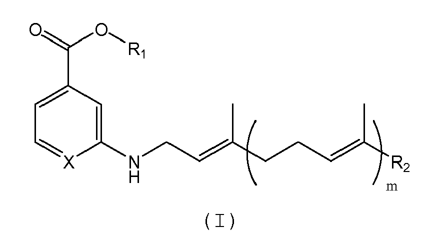 its pharmaceutically acceptable salts, prodrugs and/or solvates,
wherein
X is N or CH;
R₁ is H, C₁-C₁₂ alkyl, C₂-C₁₂ alkenyl or C₂-C₁₂ alkynyl;
R₂ is methyl or ethyl; and
m is 0, 1, 2, 3, 4, 5 or 6,
in the preparation of a medicinal product for the treatment of a disease presenting altered adiponectin levels or in which it is necessary to alter the adiponectin protein levels.

In a particular aspect of the invention, the disease is selected from the group consisting of diabetes, obesity, metabolic syndrome, arteriosclerotic cardiovascular disease, dyslipidemia and lipodystrophy.

In another aspect the present invention relates to a compound of formula (I) which is: 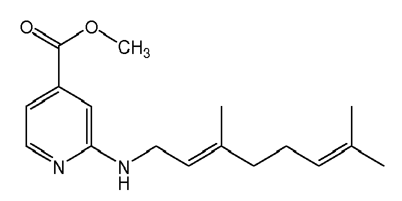 its pharmaceutically acceptable salts, prodrugs and/or solvates.

In a third aspect, the invention is aimed at a pharmaceutical composition comprising a compound of formula (I) such as the one defined previously, or its pharmaceutically acceptable salts, prodrugs or solvates, and at least one pharmaceutically acceptable carrier, adjuvant and/or vehicle.

In another aspect, the invention is aimed at a cosmetic composition comprising a compound of formula (I) such as the one defined previously or its cosmetically acceptable salts, prodrugs or solvates, and at least one pharmaceutically acceptable carrier, adjuvant and/or vehicle.

### DETAILED DESCRIPTION OF THE INVENTION

In the context of the present invention, the following terms have the meaning detailed below:

The term "C₁-C₁₂ alkyl" relates to a linear or branched hydrocarbon radical consisting of carbon and hydrogen atoms, which does not contain unsaturation, having one to twelve carbon atoms and which is joined to the rest of the molecule by a single bond. Examples of alkyl groups include but are not limited to alkyl groups such as methyl, ethyl, propyl, isopropyl, 2-methyl-1-propyl, 2-methyl-2-propyl, butyl, isobutyl, t-butyl, 2-methyl-1-butyl, 3-methyl-1-butyl, 2-methyl-3-butyl, 2,2-dimethyl-1-propyl, pentyl, 2-methyl-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, isopentyl, neopentyl, hexyl, heptyl and octyl.

The term "C₂-C₁₂ alkenyl" means a linear or branched hydrocarbon radical having one or more double carbon-carbon bonds and from 2 to 12 carbon atoms, and which is joined to the rest of the molecule by a single bond. The double bond of the alkenyl group may be conjugated or not with another unsaturated group. Suitable alkenyl groups include but are not limited to alkenyl groups such as vinyl, allyl, butenyl (1-butenyl, 2-butenyl, 3-butenyl), pentenyl (1-pentenyl, 2-pentenyl, 3-pentenyl, 4-pentenyl), hexenyl (1-hexenyl, 2-hexenyl, 3-hexenyl, 4-hexenyl, 5-hexenyl), butadienyl, pentadienyl (1,3-pentadienyl, 2,4-pentadienyl), hexadienyl (1,3-hexadienyl, 1,4-hexadienyl, 1,5-hexadienyl, 2,4-hexadienyl, 2,5-hexadienyl), 2-ethylhexenyl (2-ethylhex-1-enyl, 2-ethylhex-2-enyl, 2-ethylhex-3-enyl, 2-ethylhex-4-enyl, 2-ethylhex-5-enyl), 2-propyl-2-butenyl, 4,6-dimethyl-oct-6-enyl.

The term " C₂-C₁₂ alkynyl" means a linear or branched hydrocarbon radical having one or more triple carbon-carbon bonds and from 2 to 12 carbon atoms, and which is joined to the rest of the molecule by a single bond. The triple bond of an alkynyl group may be not conjugated or conjugated with another unsaturated group. Suitable alkynyl groups include but are not limited to alkynyl groups such as ethynyl, propynyl (e.g. 1-propynyl, 2-propynyl), butynyl (e.g. 1-butynyl, 2-butynyl, 3-butynyl), pentynyl (e.g. 1-pentynyl, 2-pentynyl, 3-pentynyl, 4-pentynyl), hexynyl (e.g. 1-hexynyl, 2-hexynyl, 3-hexynyl, 4-hexynyl, 5-hexynyl), methylpropynyl, 3-methyl-1-butynyl, 4-methyl-2-heptynyl, and 4-ethyl-2-octynyl.

Unless otherwise indicated, the compounds used in the invention are intended to include compounds that only differ in the presence of one or more isotopically enriched atoms. For example, compounds having the present structures except for the substitution of a hydrogen with deuterium or tritium, or the substitution of a carbon with a ¹³C- or ¹⁴C-enriched carbon or a ¹⁵N-enriched nitrogen are within the scope of this invention.

The term "pharmaceutically acceptable salts, solvates or prodrugs thereof" relates to salts, solvates or prodrugs which, when administered to the recipient, can provide (directly or indirectly) a compound such as the one described herein. Nevertheless, it will be observed that pharmaceutically unacceptable salts are also within the scope of the invention because they can be useful for preparing pharmaceutically acceptable salts. Salts, prodrugs and derivatives can be prepared by means of methods known in the state of the art. "Pharmaceutically acceptable" preferably relates to molecular entities and compositions which are physiologically tolerable and do not typically cause an allergic reaction or a similar unfavorable reaction, such as gastric disorders, dizziness and the like, when administered to a human. The term "pharmaceutically acceptable" preferably means that it is approved by a regulatory agency of a federal or state government or is included in the US pharmacopoeia or another generally recognized pharmacopoeia for use in animals, and more particularly in humans.

For example, the pharmaceutically acceptable salts of the compounds described previously herein are synthesized from the previously described compound containing a basic or acidic unit by means of conventional chemical methods. Such salts are generally prepared, for example, by reacting the free acidic or basic forms of these compounds with a stoichiometric amount of the suitable base or acid in water or in an organic solvent or in a mixture of both. Non-aqueous media, such as ether, ethyl acetate, ethanol, isopropanol or acetonitrile, are generally preferred. Examples of acid addition salts include mineral acid addition salts such as hydrochloride, hydrobromide, hydroiodide, sulfate, nitrate, phosphate, for example, and organic acid addition salts such as acetate, maleate, fumarate, citrate, oxalate, succinate, tartrate, malate, mandelate, methanesulfonate and p-toluenesulfonate, for example. Examples of alkaline addition salts include inorganic salts such as sodium, potassium, calcium, ammonium, magnesium, aluminium and lithium, for example, and organic alkaline salts such as ethylenediamine, e thanolamine, N,N-dialkylenethanolamine, glucamine and basic amino acid salts for example.

The term "prodrug" is defined herein as a chemical compound which has undergone a chemical derivation such as a substitution or addition of an additional chemical group in order to change (for pharmaceutical use) some of its physical and chemical properties, such as solubility or bioavailability, for example an ester or ether derived from an active compound giving an active compound *per se* after the administration to a subject. Examples of well known methods for producing a prodrug from a given active compound are known by persons skilled in the art and can be found in Krogsgaard-Larsen et al., Textbook of Drug Design and Discovery, Taylor & Francis (April 2002), for example. According to this invention, the term "solvate" is understood to mean any form of a compound of the invention having another molecule (most likely a polar solvent) bound to it through a non-covalent bond. Examples of solvates include hydrates and alcoholates, for example methanolate.

Particularly preferred prodrugs are those increasing the bioavailability of the compounds of this invention when such compounds are administered to a patient (allowing an orally administered compound to be more quickly absorbed into the blood, for example) or those increasing the distribution of the original compound to a biological compartment (the brain or the lymphatic system, for example) relating to the original species.

The compounds used in the invention may be in crystalline from, either as free compounds or as solvates (hydrates, for example) and it is understood that both forms are within the scope of the present invention. Solvation methods are generally known in the art. Suitable solvates are pharmaceutically acceptable solvates. In a particular embodiment, the solvate is a hydrate.

Salts, solvates and prodrugs can be prepared by means of methods known in the state of the art. It will be observed that pharmaceutically unacceptable salts, solvates or prodrugs are also included within the scope of the invention because they can be useful in the preparation of pharmaceutically acceptable salts, solvates or prodrugs.

The compounds of formula (I) or their salts or solvates are preferably in pharmaceutically acceptable form or in substantially pure form. A pharmaceutically acceptable form is understood, *inter alia,* as having a pharmaceutically acceptable purity level, excluding normal pharmaceutical additives such as diluents and excipients, and without including any material considered to be toxic at normal dosage levels. The purity levels for the drug are preferably above 50%, more preferably above 70%, and still more preferably above 90%. In a preferred embodiment, it is above 95% of the compound of formula (I), or of its salts, solvates or prodrugs.

The compounds used in the invention shown by the formula (I) described above can include enantiomers depending on the presence of chiral centers or isomers depending on the presence of multiple bonds (for example, Z, E). The individual isomers, enantiomers, diastereoisomers and mixtures thereof are within the scope of the present invention.

In a preferred embodiment of the invention, the radical R₁ of the compound of formula (I) used in the invention is hydrogen or C₁-C₆ alkyl, it is more preferably hydrogen or methyl.

In another preferred embodiment, m is 0 or 1.

In another preferred embodiment, the radical R₂ is methyl.

In another still more preferred embodiment, the compound of formula (I) is selected from the following compounds:

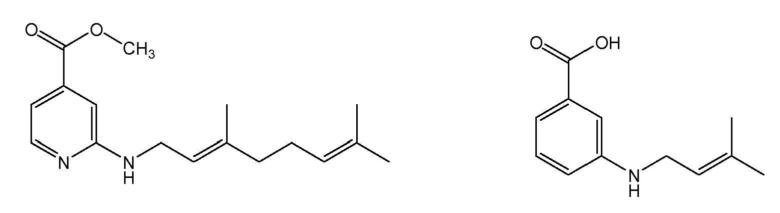

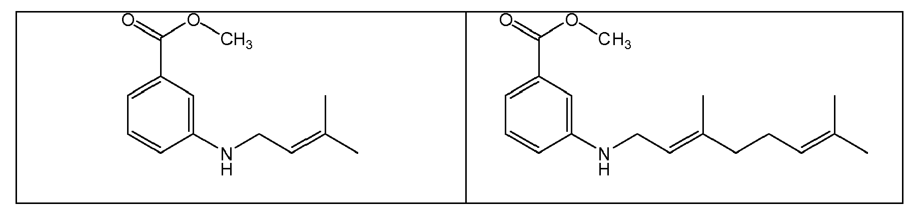

The compounds of formula (I) used in the present invention can be prepared synthetically starting from commercially available compounds; all the compounds can be synthesized by direct alkylation of anilines or pyridinylamines differently substituted with the corresponding commercially available alkyl bromides. However, a subsequent hydrolysis of the methyl ester group is required for some of the compounds.

For example, 2.5 equivalents of powder potassium carbonate are added to a solution of aniline or pyridinylamine in anhydrous (anh.) THF, and the resulting mixture is stirred for 10 minutes. 1.1 eq of the corresponding unsaturated alkyl bromide in THF are added and the resulting mixture is left stirring for more than 16-18 hours. The solvent is evaporated under reduced pressure, 1M HCl solution is added and the resulting mixture is extracted with DCM. The pooled extracts are washed with water, saturated NaCl solution and are dried (Na₂SO₄) and the solvent is evaporated to dryness. The resulting residue is purified in a flash chromatographic column using an eluent mixture as indicated for each case in the invention examples.

In an additional aspect, the present invention provides a compound of formula (I) which is: 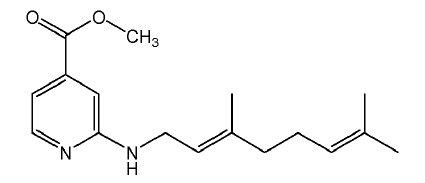 its pharmaceutically acceptable salts, prodrugs and/or solvates.

The present invention further provides pharmaceutical compositions comprising the novel compound of formula (I) of the present invention, or pharmaceutically acceptable salts, solvates or prodrugs thereof and at least one pharmaceutically acceptable carrier, adjuvant and/or vehicle, for the administration to a patient.

In a particular embodiment, for its administration in the prevention and/or treatment of diseases presenting altered adiponectin levels or in which it is necessary to alter the protein levels, the compounds of formula (I), their pharmaceutically acceptable salts, prodrugs and/or solvates will be formulated in a suitable pharmaceutical composition, in the therapeutically effective amount, together with one or more pharmaceutically acceptable carriers, adjuvants, and/or vehicles.

The term "carrier, adjuvant and/or vehicle" relates to molecular entities or substances with which the active ingredient is administered. Such pharmaceutical carriers, adjuvants or vehicles can be sterile liquids, such as waters and oils, including those of petroleum or with an animal, plant or synthetic origin, such as peanut oil, soybean oil, mineral oil, sesame oil and the like, excipients, disintegrants, wetting agents or diluents. Suitable pharmaceutical carriers are described in "Remington's Pharmaceutical Sciences" by E.W. Martin.

The pharmaceutical compositions can be administered by any suitable method of administration, for example, oral, parenteral (for example, subcutaneous, intraperitoneal, intravenous, intramuscular, etc.), rectal administration etc., typically orally due to the chronic nature of the disease to be treated.

In a particular embodiment, said pharmaceutical compositions can be in an oral administration pharmaceutical form, either in solid or liquid form. Illustrative examples of oral administration pharmaceutical forms include tablets, capsules, granulates, solutions, suspensions, etc., and can contain conventional excipients such as binders, diluents, disintegrants, lubricating agents, wetting agents, etc., and can be prepared by conventional methods. The pharmaceutical compositions can also be adapted for their parenteral administration, in the form of, for example, sterile, lyophilized products, suspensions or solutions in the suitable dosage form; in this case, said pharmaceutical compositions will include suitable excipients, such as buffers, surfactants, etc. In any case, the excipients will be chosen according to the selected administration pharmaceutical form. A review of the different pharmaceutical forms for administering drugs and of their preparation can be found in "Tratado de Farmacia Galénica", by C. Faulí i Trillo, 10th Edition, 1993, Luzán 5, S.A. de Ediciones.

For its application in therapy, the compound of formula (I) will preferably be found in a pharmaceutically acceptable or substantially pure pharmaceutical form, i.e. the compound of formula (I) has a pharmaceutically acceptable purity level excluding pharmaceutically acceptable excipients and does not include material considered to be toxic at normal dosage levels. The purity levels for a compound of formula (I) are preferably greater than 50%, more preferably greater than 70%, more preferably greater than 90%. In a preferred embodiment, they are greater than 95%.

The therapeutically effective amount of the compound of formula (I) to be administered will generally depend, among other factors, on the individual to be treated, on the severity of the disease suffered by said individual, on the chosen method of administration, etc. For this reason, the doses mentioned in this invention must only be considered as guidelines for the person skilled in the art, and the latter must adjust the doses according to the aforementioned variables. Nevertheless, a compound of formula (I) can be administered once or more times a day, for example, 1, 2, 3 or 4 times a day, in a typical total daily amount comprised between 0.1 and 1000 mg/kg of body mass/day, preferably 10 mg/kg of body mass/day.

The compound of formula (I), its pharmaceutically acceptable salts, prodrugs and/or solvates, as well as the pharmaceutical compositions containing them can be used together with other additional drugs useful for preventing and/or treating diseases presenting altered adiponectin levels or in which it is necessary to alter the adiponectin protein levels. Said additional drugs can form part of the same pharmaceutical composition or alternatively, can be provided in the form of a separate composition for its simultaneous or non-simultaneous administration with the pharmaceutical composition comprising a compound of formula (I), or a pharmaceutically acceptable salt, prodrug or solvate thereof.

Within the scope of the present invention, the expression "disease or condition presenting altered adiponectin levels or in which it is necessary to alter the adiponectin protein levels" relates to any disease, disorder or condition in which the adiponectin levels, as they can be measured in plasma, are outside the values considered to be normal and obtained as an average of the plasma adiponectin concentration in a human population that does not have these diseases, or in which the modification of the adiponectin levels causes improvements in that disease, disorder or condition. The disease is preferably selected from the group consisting of diabetes, obesity, metabolic syndrome, arteriosclerotic cardiovascular disease, dyslipidemia and lipodystrophy.

Another aspect of the present invention is a method for treating and/or preventing a disease or disorder presenting altered adiponectin levels or in which it is necessary to alter the adiponectin levels, which comprises administering to a patient who needs such treatment a therapeutically effective amount of at least one compound of formula (I) as defined above or a pharmaceutical composition thereof.

The disease or disorder is preferably selected from, but is not limited to diabetes, obesity, metabolic syndrome, arteriosclerotic cardiovascular disease, dyslipidemia and lipodystrophy.

The term "treatment" or "treat" in the context of this specification means the administration of a compound with a formulation according to the invention for preventing, alleviating or eliminating the disease or one or more symptoms associated to said disease. "Treatment" also includes preventing, alleviating or eliminating the physiological sequelae of the disease.

The term "alleviate" in the context of this invention is understood to mean any improvement of the situation of the treated patient- both subjectively (the feelings of or about the patient) and objectively (measured parameters).

The present invention is additionally explained below by means of examples. This explanation must by no means be interpreted as a limitation of the scope of the invention as it is defined in the claims.

### Examples

### Example 1. Synthesis of 2-(3,7-dimethyl-octa-2,6-dienylamino)-isonicotinic acid methyl ester (compound 1).

Potassium carbonate (2.27g; 16 mmoles) is added to a solution of 2-amino-isonicotinic acid methyl ester (1g; 6.57 mmoles) in anhydrous tetrahydrofuran (50 ml). A nitrogen atmosphere is made and after 5 minutes, a solution of geranyl bromide (1.43 ml; 7.23 mmoles) in anhydrous tetrahydrofuran (10 ml) is added. The mixture is heated at 60°C for 16 hours, after which the mixture is allowed to reach room temperature. A small amount of silica gel is added to act as the column head and the mixture is taken to dryness. It is purified by a chromatographic column using a mixture of ethyl acetate and hexane in a 1:10 ratio as the eluent, obtaining the desired product with a 10% yield and a 100% purity.
¹H-NMR (400MHz) 8.19 (dd; 1H; J = 0.6 Hz; J = 5.2 Hz; **H3**); 7.08 (dd; 1H; J = 1.4 Hz; J = 5.2 Hz; **H4**); 6.95 (dd; 1H; J = 0.8 Hz; J = 1.3 Hz; **H2**); 5.31 (dt; 1H; J = 1.2 Hz; J = 6.8 Hz; **H7**); 5.08 (dt; 1H; J = 1.3 Hz; J = 6.8 Hz; **H11**)**;** 4.62 (t; 1H; J = 4.7 Hz; **H5**); 3.91 (s; 3H; **H1**); 3.89 (d; 2H; J = 5.9 Hz; **H6**); 2.08 (m; 4H; **H10; H9**); 1.73 (s; 3H; **H8**); 4.62 (s; 3H; **H13**); 4.62 (s; 3H; **H12**)
¹³C-NMR (100MHz) 166.28 (B); 159.27 (E); 149.00 (F); 139.85 (J); 138.65 (C); 131.74 (N); 123.82 (M); 120.45 (I); 111.61 (G); 106.54 (D); 52.43 (A); 40.28 (H); 39.51 (L); 26.41 (LL); 25.66 (O); 17.69 (N); 16.39 (K)

### Example 2. Synthesis of 3-(3-methyl-but-2-enylamino)-benzoic acid (compound 2)

In a 50ml flask, 3-amino-benzoic acid methyl ester (210mg, 0.957mmoles) is dissolved in 5ml of THF, water (3ml) and methanol (2ml) are then added. Lithium hydroxide monohydrate (15 eq., 14mmoles, 603mg) is added and it is left at room temperature overnight. It is neutralized with 1M HCl up to slightly acidic pH and it is extracted with dichloromethane (3x25ml), washed with water (1x25ml), dried with anhydrous sodium sulfate, filtered and taken to dryness. It is purified by a silica gel chromatography column using dichloromethane-2% methanol as the mobile phase, obtaining the desired product with a 97% yield and a 95.5% purity.
¹H-NMR (25°C, CDCl₃, 400 MHz, ppm) 7.46 (d, 1H, J=7.8Hz); 7.34 (d, 1H, J=3.9Hz); 7.26 (t, 1H, J=7.9Hz); 6.83 (ddd, 1H, J=0.9Hz, J=2.6Hz, J=8.1Hz); 5.33 (dt, 1H, J=1.4Hz, J=6.8Hz); 3.74 (d, 2H, J=6.8Hz); 1.76 (s, 3H); 1.74 (s, 3H).
¹³C-NMR (25°C, CDCl₃, 100 MHz, ppm) 172.4; 148.3; 136.1; 130.1; 129.1; 121.0; 119.0; 118.2; 113.7; 41.9; 25.6; 18.0.

### Example 3. Synthesis of 3-(3-methyl-but-2-enylamino)-benzoic acid methyl ester (compound 3)

In a 100ml flask, acid 3-amino-benzoic acid methyl ester (500mg; 3.31mmoles) is dissolved in 25ml of anh. THF, powdered potassium carbonate (2.5eq., 8.27mmoles; 1.14g) is then added and a nitrogen atmosphere is made. The reaction is left stirring for 10 minutes and farnesyl bromide (1.1eq., 3.64mmoles; 0.42ml) dissolved in 5ml of THF is added and it is left stirring overnight at room temperature. The reaction is stopped by adding a small amount of silica gel so that it acts as a column head and it is taken to dryness. It is purified by a silica gel column chromatography using a mixture of ethyl acetate/hexane in a 1:30 ratio as the mobile phase, separating two products. The product is obtained with a 33% yield and a 96% purity.
¹H-NMR (25°C, CDCl₃, 400 MHz, ppm) 7.36 (ddd, 1H, J=1.0Hz, J=1.5Hz, J=7.6Hz); 7.27 (dd, 1H, J=1.7Hz, J=2.5Hz); 7.22 (t, 1H, J=7.9Hz); 6.77 (ddd, 1H, J=1.0Hz, J=2.6Hz, J=8.1Hz); 5.32 (dt, 1H, J=1.4Hz, J=6.8Hz); 3.89 (s, 3H); 3.72 (d, 2H, J=6.8Hz); 1.75 (s, 3H); 1.73 (s, 3H).

¹³C-NMR (25°C, CDCl₃, 100 MHz, ppm) 167.5; 148.3; 136.0; 130.9; 129.0; 121.0; 118.3; 117.3; 113.2; 51.9; 41.9; 25.6; 18.0.

### Example 4. Synthesis of 3-(3,7-dimethyl-octa-2,6-dienylamino)-benzoic acid methyl ester compound (compound 4)

In a 100ml flask, 3-amino-benzoic acid methyl ester (500mg; 3.31mmoles) is dissolved in 25ml of anh. THF, powdered potassium carbonate (2.5eq., 8.27mmoles; 1.14g) is then added and a nitrogen atmosphere is then made. The reaction is left stirring for 10 minutes and geranyl bromide (1.1eq., 3.64mmoles; 0.86ml) dissolved in 5ml of THF is added and it is left stirring overnight at room temperature. The reaction is stopped by adding a small amount of silica gel so that it acts as a column head and it is taken to dryness. It is purified by a silica gel column chromatography using a mixture of ethyl acetate/hexane in a 1:30 ratio as the mobile phase, separating two products. The product is obtained with an 8% yield and a 98.5% purity.
¹H-NMR (25°C, CDCl₃, 400 MHz, ppm) 7.36 (dt, 1H, J=1.3Hz, J=7.7Hz); 7.27 (t, 1H, J=2.0Hz); 7.22 (t, 1H, J=7.9Hz); 6.78 (ddd, 1H, J=0.9Hz, J=2.5Hz, J=8.0Hz); 5.32 (dt, 1H, J=1.2Hz, J=6.7Hz); 5.08 (dt, 1H, J=1.4Hz, J=6.8Hz); 3.89 (s, 3H); 3.74 (d, 2H, J=6.6Hz); 2.08 (m, 4H); 1.72 (s, 3H); 1.68 (s, 3H); 1.60 (s, 3H).
¹³C-NMR (25°C, CDCl₃, 100 MHz,) 167.5; 148.3; 139.5; 131.7; 130.9; 129.0; 123.8; 120.9; 118.3; 117.3; 113.3; 51.9; 41.9; 39.5; 26.4; 25.6; 17.6; 16.3.

### Example 5. Biological assays

The following compounds have been assayed:

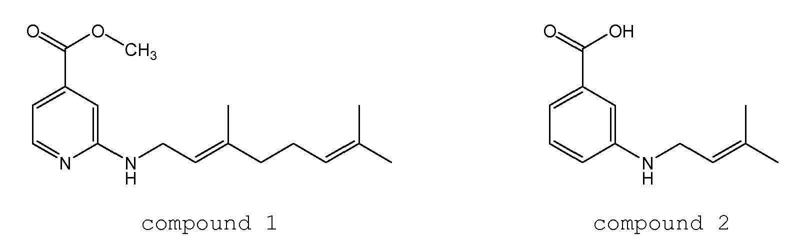

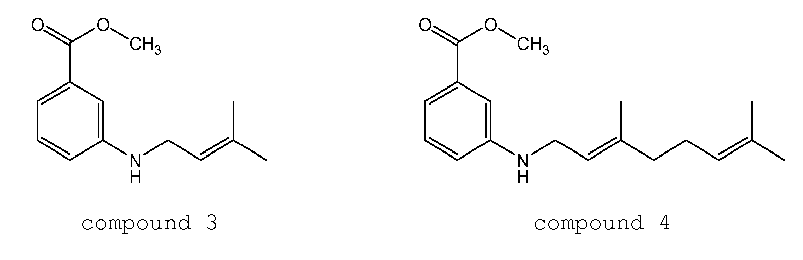

The following protocol has been followed for the purpose of analyzing the potential therapeutic activity against obesity, type 2 diabetes mellitus and atherosclerosis (ODA metabolic diseases) of the compounds 1-4:
- generating cell lines which have stably integrated human promoters of key genes in the development of ODA diseases;
- amplifying the human promoter sequence of the target genes starting from commercial human genomic DNA;
- cloning the promoter into a suitable vector which allows knowing the transcriptional activity of the promoter sequence, using the luciferase gene as a reporter gene;
- generating stable cell lines for each of the promoters cloned into the reporter vectors;
- carrying out the screening test with the compounds of the invention on the stable cell lines;
- suitably validating the effect obtained with a certain compound which can modify the transcriptional activity of the promoter.

The selected cell line is C2C12, corresponding to myoblasts from mouse muscle tissue.

Thus, on the first day, the cells are seeded in P6 (6 wells) in their culture medium. In this case, 160,000 C2C12 cells per well are seeded in complete DMEM + 10% fetal bovine serum (FBS).

On the second day, the cells are cotransfected with 2.5 µg of the plasmid containing the construct of interest (promoter + reporter gene (luciferase)) and 0.5 µg of the pH1c plasmid containing the hygromycin resistance gene. The transfection used is:
TRANSFAST (Promega): 1:3 Ratio, µg DNA: µl Transfast.
   1. The culture medium, DMEM (600 µl per well), is mixed with the DNA specified above. The reaction is incubated for 5 minutes at room temperature.
   2. The TransFast reagent is added to the mixture and a 15-minute incubation is carried out at room temperature.
   3. After this time elapses, the medium is removed from the wells and the transfection volume is added dropwise on the cells.
   4. The mixture is incubated for 1 hour at 37°C; the transfection mixture is then removed and complete medium is added.

A double positive control is always used for the transfection process, using GFP ("Green Fluorescent Protein") reporter genes and β-galactosidase, in the same conditions as the constructs of interest. These 2 reporter genes further allow estimating the efficiency of the transfection process, as described below.

On the third day, the transfection efficiency is observed:
a) through the GFP gene expression, using a fluorescence microscope: the fluorescing cells (using the corresponding filter while observing them) have correctly integrated the reporter gene, i.e., they have been correctly transfected.
b) through the β-galactosidase gene expression, with a specific stain kit which allows observing the cells under the microscope once they have been stained. The cells which have integrated the reporter gene can use the reaction substrate, generating a blue color as a product of said chemical reaction.

Both methods allow estimating what percentage of cells has been successfully transfected (those that are green or blue, according to if they have integrated GFP or β-galactosidase, respectively). By extension, and given that identical conditions are used, this successful transfection percentage can be applied in the case of the constructs of interest.

By considering 40-60% a high transfection percentage, and only if said percentage is reached, 1/100 and 1/150 passages are carried out on the cells ("dilutions"), seeding them already on new P6 plates. The amount of hygromycin added per well is 700 µg/ml (1.33 mM).

A hygromycin resistance curve with different drug concentrations has previously been made with cells seeded in P6 to choose the suitable amount of drug to be used in the screening.
Obtaining clones. In the following days, the cells are always maintained with hygromycin and are observed under the microscope to see the evolution of the possible clones. When a clone covers a ten-times magnification lens when observed under a microscope, it is considered large enough to isolate it from that plate and set up a stable cell line from it. It is important to choose clones that are not very close to one another in the well, in order to prevent the possible mixture of clones while setting up the stable cell line. The protocol which is followed to isolate each clone is described below:
1. The culture medium is removed.
2. The cells are washed twice with PBS1x.
3. The cloning rings are placed (with petroleum jelly) on each clone and they are pressed with clamps so that they are adhered through the petroleum jelly.
4. 20-30 µl of trypsin are added in each ring and the reaction is incubated for 5 minutes at 37°C. Once it has been verified that all the clone cells have been suitably trypsinized, 100 µl of culture medium are added to stop said reaction.
5. It is pipetted up and down taking care to not move the ring and the necessary volume is added to reach 1 ml of medium in P24; this added volume will be medium with hygromycin to continue with the stable cell screening.

Escalate successively from P24 to P6 and from P6 to T25. When the cells of the clone reach confluence in T25, a vial is frozen and the transcriptional activity of the promoter which they have stably integrated is measured through the luciferase reporter gene. The clones that are positive in the study will continue to be maintained in hygromycin, measuring luciferase periodically in order to control that they do not lose said activity.

The luciferase activity is measured according to the following protocol:
a) 50000 and 1200000 cells/well (and in duplicate) are seeded in a P24 plate.
b) After approximately 24 hours, the medium is removed from the cells and each well is washed with PBS 1x (because DMEM interferes in the measurement of the activity of the reporter gene)
c) 100 µl of luciferase developing reagent are added in each cell (Bright-GloTM Luciferase Assay system- Promega). This reagent carries the reporter gene substrate and luminescence is generated as the product of the enzymatic reaction. The cells which have acquired the reporter gene, and therefore the target promoter, will be luminescent upon being in the presence of said substrate. To record the measurement of luciferase activity, the plate is shaken for 30 seconds right after adding the developing reagent and after 2 minutes, the volume is collected and transferred to a 96-well plate (P96), which can be read in an illuminometer.
d) The suitable luminescence program is selected, such that the reading lasts 5 seconds per well, and the arbitrary luminescence units are recorded for each obtained clone or, in other words, for each stable cell line.

The reaction substrate is photosensitive, therefore it is important to keep the plate in darkness from the time the reagent is added until the luciferase activity is recorded or read in each experiment.
Carrying out the screening test: The clones used in the screening are: C2C12 Adipo (adiponectin promoter), C2C12 UCP3 (UCP3 promoter), C2C12 AdR1 (adiponectin receptor 1 promoter) and C2C12 Nmu (neuromedin promoter).

Before any screening experiment, the suitable number of cells for carrying out such experiments must first be provided. To that end, 2 T75 are usually seeded (with approximately 1x10⁶ cells in each bottle), such that after four days, the cells have reached confluence and are ready for the screening protocol detailed below to be carried out.
1. Day 1: 30000 cells per well are seeded in white P96 plates; these plates allow amplifying the luminescence signal and improving the reading. The cells are grown in 100 µl of hygromycin-free medium.
2. Day 2: 24 hours after the seeding, the cells are stimulated with compounds 1-4.
   Compounds 1-4 are assayed in duplicate at 1, 5 and 10 µg/ml concentrations, by means of the screening method shown in the following diagram: 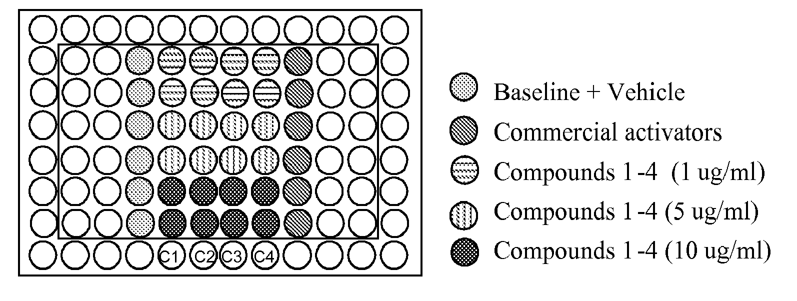 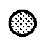 In the indicated positions, the baseline transcriptional activity of the promoters to be studied is recorded in the presence of the medium in which compounds 1-4 (vehicle) are dissolved.
   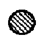 In the indicated positions, the transcriptional activity of the promoters to be studied is recorded in the presence of a known commercial activator of the target promoter. These wells have a dual function: a) they are useful as a positive control of the transcriptional activation and of the screening protocol; b) they are useful as a reference when screening those positive compounds increasing the activity of the promoters to be studied because they provide an activation ratio which is calculated as follows: by dividing the average luminescence signal increase in the presence of the commercial activator by the average luminescence signal provided by the baseline activity of the target promoter in the presence of vehicle. Rosiglitazone (RGZ) was initially chosen as the commercial activator due to the fact that it was described in the literature as an adiponectin gene transcription activator. A dose-response curve was made to establish the concentration at which the greatest transactivation was achieved, said concentration being 20 µM. RGZ was subsequently substituted with other commercial compounds such as genistein and daidzein. Both compounds are described in the literature as possible PPARgamma activators. Dose-response curves were made that were identical to those of RGZ, the concentration at which the greatest transactivation of the target promoter was obtained being 20 µM.
   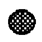 In the indicated positions, the transcriptional activity of the promoters to be studied was recorded in duplicate in the presence of 1, 5 and 10 µg/ml of compounds 1-4 of the invention in each well.
3. Day 4: 48 hours after the stimulation with compounds 1-4, the luciferase activity directed by the target promoters is developed according to the protocol described previously, introducing the following modifications:
   - Once the medium has been removed from the cells and they have been washed with PBS 1x, 40 µl of luciferase developing reagent are added in each well.
   - After 2 minutes, the reading is carried out in the same P96 plate.

### Analysis of the Results

Taking into account the diagram shown above for the screening experiments, a series of luminescence values, RLUs ("Relative Luciferase Units") are obtained which are used to calculate the aforementioned transcriptional activation ratios, which the target promoter being assayed in each experiment is in charge of. The ratio is plotted, using the luminescence signal increase in the presence of each of the compounds 1-4 which have been assayed, against the average of the luminescence signal obtained in baseline conditions (in the presence of the vehicle). Any assay in which an activation ratio ≥2 is obtained when the cells are treated with the commercial activator is considered valid. Transcriptional activity ratios greater than 1.2 are obtained for all the assayed compounds, they can therefore be considered positive compounds.

The assays were repeated at three new concentrations ranging between 0.5 and 25 µg/ml, according to the results obtained in the first assay.

The activation ratios obtained when the cells were treated with the 4 mentioned compounds together with the values for the commercial compounds are detailed below:

| | 0.5 µM | 1 µM | 5 µM | 10 µM | 25 µM |
|---|---|---|---|---|---|
| Compound 1 | 1.67 | 2.02 | 3.33 | 4.07 | 4.05 |

| | | | | | |
|---|---|---|---|---|---|
| (n=3) | | | | | |

| | 0.01 µM | 1 µM | 5 µM | 15 µM | 25 µM |
|---|---|---|---|---|---|
| Compound 2 | 1.05 | 1.69 | 2.78 | 3.43 | 3.57 |

| | | | | | |
|---|---|---|---|---|---|
| (n=6) | | | | | |

| | 0.5 µM | 1 µM | 5 µM | 10 µM | 25 µM |
|---|---|---|---|---|---|
| Compound 3 | 1.07 | 1.26 | 1.98 | 1.85 | 2.46 |

| | | | | | |
|---|---|---|---|---|---|
| (n=3) | | | | | |

| | 0.5 µM | 1 µM | 5 µM | 10 µM | 25 µM |
|---|---|---|---|---|---|
| Compound 4 | 2.32 | 2.59 | 3.53 | 3.71 | 3.47 |

| | | | | | |
|---|---|---|---|---|---|
| (n=3) | | | | | |

| Daidzein | 1 µM | 5 µM | 10 µM | 20 µM | |
|---|---|---|---|---|---|
| | 1.68 | 3.03 | 3.76 | 4.04 | |

| Genistein | 1 µM | 5 µM | 10 µM | 20 µM | |
|---|---|---|---|---|---|
| | 1.47 | 2.25 | 3.59 | 3.05 | |

Once it has been shown that the compounds are positive, a dose-response (µM) curve is then made to know: a) the concentration at which the maximum transactivation of the promoter is obtained in the presence of that compound and b) the concentration at which 50% transactivation, over the maximum observed (EC₅₀), is obtained. The results obtained are detailed below:

| | 50 µM | 100 µM | 200 µM | | |
|---|---|---|---|---|---|
| Compound 1 | 3.92 | 3.06 | 2.57 | | |

| | 50 µM | 200 µM | 400 µM | | |
|---|---|---|---|---|---|
| Compound 2 | 3.66 | 3.04 | 1.81 | | |

| | 50 µM | 100 µM | 200 µM | | |
|---|---|---|---|---|---|
| Compound 3 | 2.61 | 2.34 | 1.92 | | |

| | 50 µM | 100 µM | 200 µM | | |
|---|---|---|---|---|---|
| Compound 4 | 3.28 | 2.53 | 1.78 | | |

| Daidzein | 50 µM | 100 µM | | | |
|---|---|---|---|---|---|
| | 4.05 | 3.71 | | | |

| Genistein | 50 µM | 100 µM | 200 µM | | |
|---|---|---|---|---|---|
| | 1.97 | 1.75 | 1.59 | | |

## Claims

1. The use of a compound of formula (I): 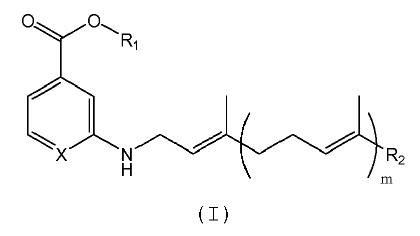 its pharmaceutically acceptable salts, prodrugs and/or solvates, wherein
X is N or CH;
R₁ is H, C₁-C₁₂ alkyl, C₂-C₁₂ alkenyl or C₂-C₁₂ alkynyl;
R₂ is methyl or ethyl; and
m is 0, 1, 2, 3, 4, 5 or 6,
in the preparation of a medicinal product for the treatment of a disease presenting altered adiponectin levels or in which it is necessary to alter the adiponectin levels.

2. The use according to claim 1, wherein the disease mediated by the reduction of the adiponectin protein expression is selected from the group consisting of diabetes, obesity, metabolic syndrome, arteriosclerotic cardiovascular disease, dyslipidemia and lipodystrophy.

3. The use according to claim 1 or 2, wherein R₁ is hydrogen or C₁-C₆ alkyl.

4. The use according to claim 3, wherein R₁ is hydrogen or methyl.

5. The use according to any of claims 1 to 4, wherein m is 0 or 1.

6. The use according to any of claims 1 to 5, wherein R₂ is methyl.

7. The use according to any of claims 1 to 6, wherein the compound of formula (I) is selected from:

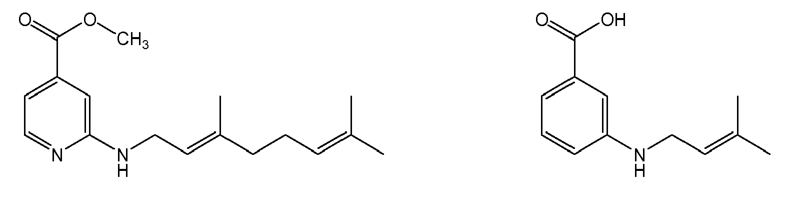

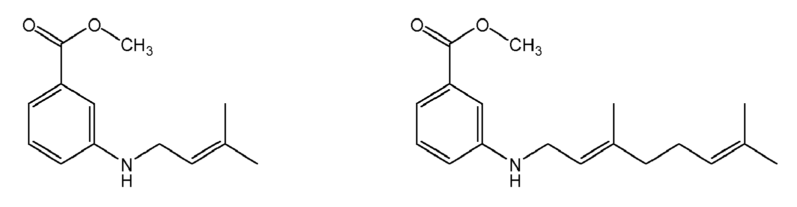

8. A compound of formula (I) which is: 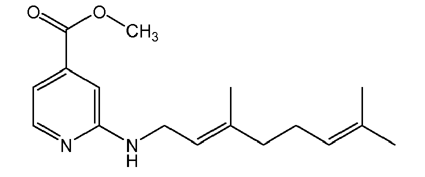 its pharmaceutically acceptable salts, prodrugs and/or solvates.

9. A pharmaceutical composition comprising a compound of formula (I) as it is defined in claim 8, or its pharmaceutically acceptable salts, prodrugs or solvates, and at least one pharmaceutically acceptable carrier, adjuvant and/or vehicle.

10. A cosmetic composition comprising a compound of formula (I) as it is defined in claim 8, or its cosmetically acceptable salts, prodrugs or solvates, and at least one cosmetically acceptable carrier, adjuvant and/or vehicle

11. A compound of formula (I) as it is defined in claim 8, for its use as a medicinal product.
